# EUROPÄISCHE PATENTSCHRIFT

(11) **EP 1 919 528 B1**
(45) Veröffentlichungstag und Bekanntmachung des Hinweises auf die Patenterteilung: **18.05.2011**
(21) Anmeldenummer: 06791729.4
(22) Anmeldetag: 30.08.2006
(51) Int. Cl.: A61L 15/26, A61L 15/42

(54) **FORMKÖRPER ZUR MEDIZINISCHEN BEHANDLUNG VON WUNDEN**
MOULDED BODY FOR MEDICALLY TREATING WOUNDS
CORPS MOULE PERMETTANT DE TRAITER DES BLESSURES

(30) Priorität: 01.09.2005 DE 102005042707
(43) Veröffentlichungstag der Anmeldung: 14.05.2008
(73) Patentinhaber: PolyMedics Innovations GmbH, 70794 Filderstadt (DE)
(72) Erfinder: HIERLEMANN, Helmut, 73033 Göppingen (DE); PLANCK, Heinrich, 72622 Nürtingen (DE); UHLIG, Christian, 70563 Stuttgart (DE)
(74) Vertreter: Patentanwälte Ruff, Wilhelm, Beier, Dauster & Partner
(86) Internationale Anmeldenummer: PCT/EP2006/008470
(87) Internationale Veröffentlichungsnummer: WO 2007/025729

(56) Entgegenhaltungen:
- EP-A- 0 908 482
- EP-A- 1 064 958
- WO-A-01/02033
- DE-A1- 10 041 684

## Beschreibung

Die vorliegende Erfindung betrifft einen resorbierbaren und porösen Formkörper zur medizinischen Behandlung von Wunden, insbesondere als Wundauflage, ein Verfahren zu dessen Herstellung sowie verschiedene Verwendungen des Formkörpers.

In den letzten Jahren ist der klinische Bedarf an Behandlungsmöglichkeiten von akuten und chronischen Wunden, insbesondere durch Verschluss mit geeigneten Abdeckungsmaterialien, stetig gestiegen. Gerade chronische Wunden, beispielsweise venöse Geschwüre, diabetisch induzierte Ulcera der Extremitäten sowie Druckgeschwüre, deren Prävalenz in westlichen Industrieländern ständig zunimmt, bedeuten für den Patienten häufig die Gefahr einer schweren Infektion, einer langen Hospitalisierungszeit sowie möglicherweise sogar eine Amputation der betroffenen Gliedmaßen. Aber auch die Behandlung von akuten Wunden, beispielsweise Verbrennungswunden, stellt hohe Anforderungen an die Behandlung der traumatisierten Körperregionen, um unerwünschte Risiken, insbesondere Wasserverlust, Temperaturabfall und eine erhöhte Infektionsgefahr, zu vermeiden.

Zu diesem Zweck wurden eine Vielzahl von bioverträglichen Verbandmaterialien auf der Basis natürlicher und synthetischer Stoffe entwickelt. Die derzeit kommerziell erhältlichen Verbandmaterialien bestehen vorwiegend aus nicht resorbierbaren synthetischen Polymeren, beispielsweise Silikon, Polyurethan (PUR), Polypropylen (PP), Polyethylenterephtalat (PET), Polyamid (PA) sowie Polytetrafluorethylen (PTFE), oder aus resorbierbaren Polymeren natürlichen Ursprungs, insbesondere Collagen, Hyaluronsäure, Cellulose, Polylactid oder Polyglycolid.

Die Nachteile der bekannten Materialien bestehen insbesondere in ihrer unvollständigen oder zumindest doch sehr langsamen Resorbierbarkeit im Körper des Patienten. Außerdem kann es zu unerwünschten Adhäsionen der Verbandmaterialien mit dem Körpergewebe kommen, die bei einem oberflächlichen Verbandwechsel eine schmerzhafte Traumatisierung beim Patienten auslösen können. Außerdem kann es aufgrund der Ablösung frisch epithelisierter Hautareale zu Narbenbildungen und damit zu einem unbefriedigenden kosmetischen Ergebnis kommen.

Aus der DE 100 41 684 A1 ist bereits ein leistungstarkes Beschichtungspolymer auf der Basis von Lactid, Trimethylencarbonat und ε-Caprolacton bekannt, welches insbesondere zur Behandlung von Verbrennungswunden ersten und zweiten Grades verwendet wird. Das Beschichtungsmaterial weist beispielsweise vorteilhafte Degradations- und Resorptionszeiten in vivo auf, so daß insbesondere eine Anhäufung von Fremdmaterialien und damit verbundene nicht vorhersehbare Nebenwirkungen im Körper des Betroffenen vermieden werden.

Bei Ulcera, d.h. großflächigen, tiefen und stark nässenden Wunden stellt sich jedoch häufig das Problem, daß sich größere Mengen an Wundwasser im Wundbett bzw. -milieu ansammeln, wodurch aufgrund des im Wundwasser angereicherten infektiösen Materials die Gefahr einer sich ausbreitenden Infektion steigt. Dies impliziert unkalkulierbare Behandlungsrisiken für den Patienten und ist daher aus medizinischer Sicht nicht erwünscht. Außerdem besteht bei insbesondere stark exsudierenden Körpergebieten grundsätzlich eine erhöhte Infektionsgefahr mit weiteren Erregern.

Die Erfindung stellt sich die Aufgabe, einen Formkörper aus einem bioverträglichen Polymer bereitzustellen, der die aus dem Stand der Technik bekannten Probleme überwindet und insbesondere eine schnelle Abführung großer Mengen von infektiösem Wundwasser erlaubt und somit eine leistungsstarke Behandlungs- bzw. Versorgungsmöglichkeit von großflächigen, tiefen und stark nässenden Wunden, beispielsweise bei Ulcera und/oder tiefdermalen Verbrennungen, insbesondere Verbrennungen zweiten und/oder dritten Grades, ermöglicht.

Diese Aufgabe wird gelöst durch einen resorbierbaren und porösen Formkörper zur medizinischen Behandlung von Wunden, insbesondere als Wundauflage, dadurch gekennzeichnet, daß er als Schaumstruktur vorliegt, die ein Co- und/oder Terpolymer auf das Basis der Monomere Lactid, Trimethylencarbonat, ε-Caprolacton und/oder Dioxan-2-on umfasst. Bevorzugt handelt es sich bei dem Monomer Dioxan-2-on um p-Dioxan-2-on.

Vorzugsweise handelt es sich bei dem resorbierbaren und porösen Formkörper um eine Schaumstruktur, die ein Terpolymer auf der Basis der Monomere Lactid, Trimethylencarbonat und ε-Caprolacton umfaßt, wobei die Schaumstruktur vorzugsweise aus diesem Terpolymer besteht. Vorzugsweise weist das Terpolymer einen Gehalt an Lactid von höchstens 85 Gew.-%, insbesondere höchstens 80 Gew.-%, an Trimethylencarbonat im Bereich von 5 bis 20 Gew.-%, insbesondere 10 bis 20 Gew.-%, und an ε-Caprolacton im Bereich von 5 bis 20 Gew.-%, insbesondere 5 bis 15 Gew.-%, auf. Bezüglich weiterer Einzelheiten wird auf die DE 100 41 684 A1 verwiesen, deren Offenbarungsgehalt von der vorliegenden Erfindung ausdrücklich miterfaßt sein soll.

Die Monomere Lactid/Trimethylencarbonat/ε-Caprolacton können insbesondere in Verhältnissen im Bereich von 88/8/4 bis 70/20/10 Ges.-%, bezogen auf das Gesamtgewicht des Terpolymers vorliegen.

In einer anderen Ausführungsform liegt der Formkörper als eine Schaumstruktur vor, die ein Copolymer auf der Basis der Monomere

Lactid und Trimethylencarbonat umfaßt. Vorzugsweise besteht die Schaumstruktur aus diesem Copolymer.

Die Lactidkomponente im Co- und/oder Terpolymer kann L-Lactid, D-Lactid und/oder DL-Lactid sein, wobei DL-Lactid bevorzugt ist.

Das Co- und/oder Terpolymer kann ab initio ein Molekulargewicht im Bereich von 80.000 bis 400.000 Dalton, insbesondere von 90.000 bis 250.000 Dalton, aufweisen. Im sterilisierten Zustand, insbesondere nach einer Gammabestrahlung, beispielsweise von 25 kGy, weist das Co- und/oder Terpolymer vorzugsweise ein Molekulargewicht von 50.000 bis 150.000 Dalton auf.

Weiterhin kann das Co- und/oder Terpolymer ab initio eine inhärente Viskosität von 0,8 bis 2,5 dl/g, insbesondere von 1,0 bis 2,0 dl/g, aufweisen, bezogen auf eine 0,1 %ige Lösung des Co- und/oder Terpolymers in Chloroform bei 25°C. Nach einer Sterilisierung, insbesondere nach einer Gammastrahlenbehandlung, beispielsweise von 25 kGy, weist das Co- und/oder Terpolymer mit Vorteil eine inhärente Viskosität von 0,6 bis 1,2 dl/g auf, bezogen auf eine 0,1 %ige Lösung des Co-und/oder Terpolymers in Chloroform bei 25 °C.

In einer besonders bevorzugten Ausführungsform des erfindungsgemäßen Formkörpers liegt die Schaumstruktur in Form eines Strukturschaums mit einem Gerüst vor, das Hohlräume bzw. Kavitäten umfaßt. Die Schaumstruktur, insbesondere der Strukturschaum, weist vorzugsweise eine Über- und eine Unterstruktur auf, wobei die Unterstruktur dadurch gebildet ist, daß das Material des Gerüstes selbst eine Schaumstruktur besitzt. Die Schaumstruktur, insbesondere in der Form eines Strukturschaums, eignet sich in hervorragender Weise für die Aufnahme von Wundwasser nach Auflage auf das Wundbett.

Gemäß einer weiteren besonders bevorzugten Ausführungsform des erfindungsgemäßen Formkörpers weist die Überstruktur der Schaumstruktur, insbesondere des Strukturschaums, eine Porengröße (Porenweite) im Bereich von 50 bis 800 µm, insbesondere im Bereich größer als 50 bis 80 µm, vorzugsweise im Bereich von 80 bis 500 µm, auf. Die Unterstruktur der Schaumstruktur, insbesondere des Strukturschaums, zeichnet sich mit Vorteil dadurch aus, daß sie eine Porengröße im Bereich von 0,1 bis 50 µm, insbesondere im Bereich von 0,5 bis 30 µm, aufweist. Die Porengrößen der Schaumstruktur weisen insbesondere eine ungleichmäßige Verteilung auf. Die deutlich größeren Poren der Überstruktur bewirken mit Vorteil eine schnelle und effektive Aufnahme des Wundwassers bzw. Exsudats. Auf diese Weise wird im Exsudat befindliches infektiöses Material schnell aus dem Wundbereich entfernt und die Wunde somit gesäubert. Dies ist besonders vorteilhaft für eine schnelle Wundheilung.

Weiterhin ist es bevorzugt, wenn die Poren der Schaumstruktur, insbesondere des Strukturschaums, zumindest teilweise, vorzugsweise vollständig, interkonnektierend miteinander verbunden sind. Die Poren sind insbesondere ungleichmäßig in der Schaumstruktur verteilt. Die Poren können unterschiedliche Formen aufweisen. Beispielsweise kann es sich bei den Poren um abgerundete Poren handeln. Die Poren können weiterhin eckig ausgebildet sein. Die Gesamtporosität des Formkörpers beträgt mit Vorteil 80 bis 96 %, insbesondere 85 bis 96 %, vorzugsweise 90 bis 96 %. Durch die außerordentlich hohe Gesamtporosität des erfindungsgemäßen Formkörpers steht ein großes Volumen für die Aufnahme von Wundwasser aus dem Wundbereich zur Verfügung. Insbesondere kann aufgrund der großen Gesamtporosität des erfindungsgemäßen Formkörpers auch nachfließendes Wundwasser aus dem Wundbereich abgeführt werden. Außerdem erlaubt eine hohe Porosität insbesondere in Kombination mit optimierten Porengrößen eine Vaskularisierung des Formkörpers und damit eine ausreichende Versorgung der Wundstelle mit Nährstoffen und Sauersoff.

In einer bevorzugten Ausführungsform weist der Formkörper eine Aufnahmekapazität für Flüssigkeiten auf, welche dem 7- bis 15-fachen, insbesondere 7- bis 12-fachen, seines Eigengewichtes entspricht. Bei den Flüssigkeiten handelt es sich vorzugsweise um Wasser oder um wässrige Flüssigkeiten, insbesondere um Körper- und/oder Gewebeflüssigkeiten. Besonders bevorzugt handelt es sich bei den Flüssigkeiten um Wundwasser.

Gemäß einer weiteren Ausführungsform weist der Formkörper einen Glasumwandlungspunkt (Tg) im Bereich von 10 bis 60°C, vorzugsweise im Bereich von 25 bis 37 °C, auf. Hiermit ergeben sich besondere Vorteile für den Einsatz des Formkörpers beim Menschen, dessen Körpertemperatur in diesem Bereich liegt.

Bevorzugt weist der erfindungsgemäße Formkörper eine Dichte von maximal 0,3 g/cm³, vorzugsweise von weniger als 0,25 g/cm³, auf, wobei die untere Grenze der Dichte bei ca. 0,05 g/cm³ liegt.

Erfindungsgemäß ist es weiterhin bevorzugt, daß der Formkörper plastisch verformbar, insbesondere komprimierbar, dehnbar und/oder biegbar, ist. Mit Vorteil ist der erfindungsgemäße Formkörper insbesondere nach Auflage auf eine Körperstelle des Patienten infolge der Erwärmung auf dessen Körpertemperatur zunehmend plastisch verformbar, bis er einen fast fließfähigen Zustand erreicht. In einem solchen Zustand kann sich der Formkörper an unterschiedliche Oberflächenprofile, insbesondere an unterschiedliche Körperareale, anpassen.

Weiterhin kann der Formkörper bei Körpertemperatur eine Dehnung von 20 bis 150% aufweisen, wobei die Dehnung im Wesentlichen von den Anteilen der verwendeten Monomere im Co- und/oder Terpolymer abhängig ist. Bevorzugt beträgt das Elastizitätsmodul des Formkörpers bei Körpertemperatur weniger als 2.000 N/mm², vorzugsweise weniger als 1.000 N/mm². Die Reißdehnung des erfindungsgemäßen Formkörpers ist ebenfalls abhängig von den Anteilen der verwendeten Monomere im Co- und/oder Terpolymer.

Der erfindungsgemäße Formkörper zeichnet sich vorteilhaft dadurch aus, daß er schneidbar ist, wobei er bevorzugt bei einer Temperatur unterhalb der Glastemperatur, vorzugsweise unterhalb von 35 °C, des Co- und/oder Terpolymers schneidbar ist.

In einer bevorzugten Ausführungsform weist der erfindungsgemäße Formkörper eine Schichtdicke von 300 µm bis 30 cm auf. Der Formkörper kann erfindungsgemäß, insbesondere nach seiner Herstellung, als Blockstruktur, insbesondere mit einer Schichtdicke zwischen 5 und 30 cm, vorliegen. Durch die schneidbaren Eigenschaften des Formkörpers kann er abhängig vom Anwendungsgebiet in der gewünschten zugeschnittenen Form bereitgestellt werden.

Unter den möglichen Ausgestaltungsformen des erfindungsgemäßen Formkörpers sind Flächengebilde, insbesondere Flachstrukturen, besonders bevorzugt. Ein als Flächengebilde, insbesondere als Flachstruktur, vorliegender Formkörper zeichnet sich bevorzugt dadurch aus, daß er eine Schichtdicke zwischen 300 µm und 5 cm aufweist. Bevorzugt weist der erfindungsgemäße Formkörper eine Schichtdicke im Bereich größer als 500 µm, insbesondere im Bereich größer als 500 bis 900 µm, vorzugsweise im Bereich von 900 µm bis 3 mm, auf. Die große Schichtdicke des Formkörpers führt in Kombination mit der großen Porenweite der Überstruktur der Schaumstruktur, insbesondere des Strukturschaums, zu einer schnellen Aufnahme großer Mengen an infektiösem Exsudat aus dem Wundbereich und damit zu einer schnellen und wirkungsvollen Wundsäuberung, was sich insbesondere in einer zügigen Wundheilung manifestiert.

Besonders vorteilhaft kann sich der erfindungsgemäße Formkörper dadurch auszeichnen, daß er im Körper des Patienten vollständig resorbierbar ist, wodurch insbesondere eine gute Bioverträglichkeit beim Einsatz in Mensch und/oder Tier erreicht wird. Der Abbau des erfindungsgemäßen Polymers erfolgt in vivo durch Stoffwechselvorgänge, an welchen insbesondere Körper- und Gewebeflüssigkeiten beteiligt sind. Durch Hydrolyse wird die Polymerkette in kleinere und insbesondere leichter lösliche Fragmente gespalten. Die Bruchstücke werden gegebenenfalls enzymatisch weiter abgebaut, wobei die Endprodukte Kohlendioxid und Wasser darstellen. Für eine gute Bioverträglichkeit ist es weiterhin entscheidend, daß während des Abbauvorganges keine toxischen Stoffwechselzwischenprodukte gebildet werden. Die im erfindungsgemäßen Formkörper verwendeten Monomere zeichnen sich durch gute Verträglichkeit und Vermeidung toxischer Reaktionen im Körper aus.

Erfindungsgemäß ist es weiterhin vorteilhaft, wenn der Formkörper frei von Weichmachern ist, insbesondere wenn dem Co- und/oder Terpolymer des Formkörpers kein Weichmacher zugesetzt ist. Auf diese Weise können die günstigen physiologischen Eigenschaften des Formkörpers weiter erhöht werden. Die Weichheit und plastische Verformbarkeit des Formkörpers sind durch die molekulare Zusammensetzung des Co- und/oder Terpolymers bedingt. Insbesondere erhöht ein höherer Gehalt an ε-Caprolacton und Trimethylencarbonat die Weichheit des Co- und/oder Terpolymers. Ein hoher Gehalt an Lactid begünstigt die Härte und Steifheit des Co- und/oder Terpolymers.

Gemäß einer weiteren Ausführungsform weist der erfindungsgemäße Formkörper eine Degradationszeit in vivo von 20 bis 35 Tagen auf. Seine Resorptionszeit in vivo kann 70 bis 120 Tage, insbesondere 80 bis 100 Tage, betragen, wobei die Resorptionszeit des Formkörper vorzugsweise nach 90 Tagen abgeschlossen ist.

Während des Abbaus des erfindungsgemäßen Formkörper ergibt sich mit Vorteil ein leicht saures Milieu von etwa pH 4,5 bis 6, insbesondere von etwa pH 5. Dies ist ein physiologisch akzeptabler pH-Bereich, der den Bedingungen des menschlichen Körpers, insbesondere der Hautoberfläche, entspricht. Dieser pH-Bereich wirkt zudem antimikrobiell, insbesondere bakterizid, und wundstimulierend, was für die Wundheilung vorteilhaft ist.

Erfindungsgemäß kann es vorgesehen sein, daß der Formkörper einen Gehalt an freien Monomeren von 0 bis 10 Gew.-%, insbesondere 0,1 bis 10 Gew.-%, vorzugsweise 1 bis 10 Gew.-%, bezogen auf das Gewicht des Co- und/oder Terpolymers, aufweist.

In einer weiteren Ausführungsform ist das Co- und/oder Terpolymer des erfindungsgemäßen Formkörpers mit einem Füllmaterial dotiert, wobei das Füllmaterial zumindest teilweise die Poren der Schaumstruktur, insbesondere die Poren der Überstruktur, auskleidet. Mit Vorteil liegt das Füllmaterial in Pulverform vor, wobei das Pulver vorzugsweise eine Korngröße von 20 bis 500 µm aufweist. Auf diese Weise kann die Stabilität des Formkörpers in wirkungsvoller Weise erhöht werden. Als Füllmaterialien kommen anorganische Stoffe, insbesondere Natrium- und/oder Calcium-Phosphate, beispielsweise Tricalciumphosphat (TCP) oder Hydroxylapatit, in Frage, wobei Tricalciumphosphat besonders bevorzugt ist. Als weitere Füllmaterialien können natürliche Polymere, insbesondere Proteine, beispielsweise Collagen, in Betracht kommen. Bevorzugt handelt es sich bei dem Füllmaterial um ein synthetisches Polymer, wobei Polyvinylalkohol (PVA) insbesondere wegen seiner sehr guten Bioverträglichkeit besonders bevorzugt ist.

Die Dotierung des Formkörpers mit einem Füllmaterial ist besonders bevorzugt, wenn eine Verstärkung der Struktur des Formkörpers und eine Verringerung seines Dehnungsvermögens, gleichzeitig aber eine schnelle und wirkungsvolle Aufnahme größerer Mengen an exsudierter Flüssigkeit aus dem Wundbereich erwünscht ist. So kann in manchen Fällen eine Dehnung von 3 bis 10% wünschenswert sein. Zu diesem Zweck kann der Formkörper mit einem Füllmaterial, insbesondere mit Tricalciumphosphat (TCP), dotiert sein. Das Co- und/oder Terpolymer des erfindungsgemäßen Formkörpers kann einen Gehalt an Füllmaterial im Bereich zwischen 10 und 300 Gew-%, vorzugsweise zwischen 20 und 100 Gew-%, bezogen auf das Gesamtgewicht des Formkörpers, aufweisen.

In einer anderen Ausführungsform kann es vorgesehen sein, daß das Co- und/oder Terpolymer mit Kombinationen von Füllmaterialien dotiert ist, wobei bezüglich der Füllmaterialien insbesondere auf die obige Beschreibung verwiesen wird.

Mit Vorteil enthält der Formkörper medizinisch wirksame Substanzen, insbesondere Impfstoffe, Antiseptika, Antibiotika, Wachstumsfaktoren oder ähnliches. So ist es beispielsweise besonders bevorzugt, das im Wundwasser befindliche infektiöse Material durch im Formkörper enthaltene Antiseptika und/oder Antibiotika abzutöten. Dies unterstützt und fördert den Wundheilungsprozeß.

Weiterhin kann der erfindungsgemäße Formkörper insbesondere frei von Zellen oder Zellkulturen sein.

Die Erfindung umfaßt ferner ein Verfahren zur Herstellung eines resorbierbaren und porösen Formkörpers, insbesondere eines Formkörpers gemäß der vorliegenden Erfindung, zur medizinischen Behandlung, insbesondere als Wundauflage, umfassend die Schritte:
- Herstellung einer Lösung eines Co- und/oder Terpolymers in einem ersten Lösungsmittel,
- Herstellung einer Suspension durch Zugabe eines in dem ersten Lösungsmittel nicht löslichen Stoffes zur Lösung,
- Abkühlung und Verfestigung der Suspension,
- Ausfällung des Co- und/oder Terpolymers und Herauslösung des Stoffes mit einem mit dem ersten Lösungsmittel mischbaren zweiten Lösungsmittel, das kein Lösungsmittel für das Co- und/oder Terpolymer ist.

In einer Ausführungsform des erfindungsgemäßen Verfahrens wird als erstes Lösungsmittel mindestens ein organisches, insbesondere wasserlösliches, Lösungsmittel verwendet, wobei das Lösungsmittel vorzugsweise ohne weitere Schwierigkeiten eingefroren werden kann. Als geeignete Lösungsmittel werden insbesondere Dimethylsulfoxid (DMSO), Dimethylacetamid (DMA), Dimethylformamid (DMF), Tetrahydrofuran (THF), Dioxan oder Mischungen davon verwendet, wobei Dimethylsulfoxid (DMSO) besonders bevorzugt ist. Vorzugsweise wird als zweites Lösungsmittel Wasser verwendet. Erfindungsgemäß kann es besonders vorteilhaft sein, wenn zur Herstellung des Formkörpers Dimethylsulfoxid (DMSO) als erstes Lösungsmittel und Wasser als zweites Lösungsmittel verwendet wird.

In einer Weiterführung des erfindungsgemäßen Verfahrens wird als Stoff ein feinteiliger Stoff, vorzugsweise ein fester Stoff, hinzugegeben. Mit Vorteil weist der Stoff eine Korngröße von 50 bis 500 µm, insbesondere von 80 bis 500 µm, vorzugsweise eine mittlere Korngröße von ca. 200 µm, auf. Vorteilhafterweise wird als Stoff ein hydrophiler bzw. wasserlöslicher Stoff verwendet, der sich in Wasser oder in einem wäßrigen System unter möglichst geringer Energiefreisetzung (geringe Exothermie) löst. Als geeignete Stoffe kommen insbesondere Salze oder organische Verbindungen, beispielsweise Harnstoff oder Zitronensäure, in Betracht. Bevorzugt handelt es sich bei dem Stoff um Zucker. Bei den Zuckern kann es sich insbesondere um Mono- oder Disaccharide, beispielsweise um Glukose, Fruktose, Dextrose, Maltose, Lactose oder Saccharose, handeln. Weiterhin kann es erfindungsgemäß vorgesehen sein, dass es sich bei den Zuckern um Polysaccharide, insbesondere um Stärke, Alginate oder Chitosan, handelt. Durch die Wahl der Korngröße des Stoffes kann in besonders vorteilhafter Weise die Porengröße des Formkörpers und somit insbesondere dessen Aufnahmekapazität für das Wundwasser beeinflußt werden.

Gegebenenfalls kann vor, während oder nach der Stoffzugabe ein Füllmaterial hinzugegeben werden, das in dem zweiten Lösungsmittel nicht löslich ist. Vorzugsweise handelt es sich bei dem Füllmaterial um ein synthetisches Polymer, insbesondere um Polyvinylalkohol (PVA). Der Polyvinylalkohol (PVA) besitzt vorzugsweise ein Molekulargewicht von mehr als 10.000 Dalton (10 kDa), damit er in Wasser nicht löslich ist. Bezüglich weiterer Merkmale des Füllmaterials wird auf die bisherige Beschreibung verwiesen.

Gemäß einer weiteren Ausführungsform des erfindungsgemäßen Verfahrens wird die nach Zugabe des Stoffes zu der Lösung des Co- und/oder Terpolymers erhaltene Suspension, welche gegebenenfalls noch ein Füllmaterial aufweist, bei einer Temperatur zwischen -10 und -30 °C, insbesondere bei einer Temperatur von ca. -20 °C, verfestigt. Dies ist besonders vorteilhaft, da auf diese Weise eine Versprödung des Co- und/oder Terpolymers vermieden werden kann und insbesondere seine Weiterverarbeitbarkeit erleichtert wird. Bevorzugt wird die Ausfällung des Co- und/oder Terpolymers sowie die Herauslösung des Stoffes in einem wässrigen Fällbad, insbesondere in einem solchen mit einer Wassertemperatur zwischen 20 und 30 °C, vorgenommen. Danach erfolgt eine Vakuumtrocknung. Die Partikel des im Auswaschlösungsmittel, insbesondere Wasser, löslichen Stoffes bilden nach der Herauslösung die Hohlräume der Überstruktur der Schaumstruktur, insbesondere des Strukturschaums. Bei der anschließenden Vakuumtrocknung bilden sich die Poren der Unterstruktur der Schaumstruktur, insbesondere des Strukturschaums.

Der gemäß der Erfindung hergestellte Formkörper kann mit üblichen physikalischen und/oder chemischen Methoden sterilisiert werden. Ein mögliches Sterilisationsverfahren umfaßt die Behandlung mit Gammastrahlen. Ein anderes Verfahren zur Sterilisierung des Formkörpers zu medizinischen Zwecken umfaßt die Verwendung von Ethylenoxid.

Weiterhin sind auch alle Formkörper Gegenstand der vorliegenden Erfindung, die nach dem erfindungsgemäßen Verfahren, insbesondere nach einem der oben beschriebenen Ausführungsformen, hergestellt bzw. herstellbar sind.

Der erfindungsgemäße Formkörper eignet sich in vorteilhafter Weise zur Verwendung als Wundabdeckungsmaterial bei der medizinischen Behandlung von Mensch oder Tier. Gemäß einer weiteren bevorzugten Ausführungsform eignet sich der Formkörper auch als Hautersatzmaterial, beispielsweise als künstlicher epidermaler und/oder dermaler Hautersatz, bei der medizinischen Behandlung von Mensch und/oder Tier, insbesondere bei Verbrennungen, vorzugsweise bei Verbrennungen dritten Grades. Weiterhin eignet sich der Formkörper zur Verwendung zur Adhäsionsprophylaxe bei der medizinischen Behandlung von Mensch und/oder Tier. In einer weiteren Ausführungsform eignet sich der Formkörper zur Verwendung als Matrix für Zellkulturen, insbesondere auf dem Gebiet der regenerativen Medizin (Tissue Engineering).

Wie bereits erwähnt und beschrieben, eignet sich der erfindungsgemäße Formkörper insbesondere zur Behandlung von großflächigen, tiefen und stark nässenden Wunden, insbesondere von Ulcera und/oder tiefdermalen Verbrennungswunden, beispielsweise von Verbrennungen zweiten und/oder dritten Grades. Ein exsudierendes Wundmilieu induziert gewöhnlich bereits das Vorliegen einer Infektion. Stark exsudierende Wunden, insbesondere Ulcera und/oder tiefdermale Verbrennungswunden, lösen bei den Betroffenen in besonderem Maße einen hohen Leidensdruck aus und bedeuten häufig nicht antizipierbare Behandlungsrisiken, insbesondere Infektionsausbreitungen und/oder eine Infektion mit weiteren Erregern. Dies geht insbesondere mit einem längeren Krankenhausaufenthalt und steigenden Behandlungskosten einher. Der erfindungsgemäße Formkörper ermöglicht durch seine Ausgestaltung als Schaumstruktur, insbesondere in Kombination mit den großen Porenweiten der Überstruktur sowie der großen Schichtdicke, eine schnelle Aufnahme großer Mengen von Wundwasser bzw. Exsudat und bewirkt somit eine wirkungsvolle Wundsäuberung. Auf diese Weise wird die Gefahr einer Infektionsausbreitung sowie die Möglichkeit einer Kontaminierung mit weiterem infektiösen Material im Wundbereich drastisch verringert.

Figurenbeschreibung:

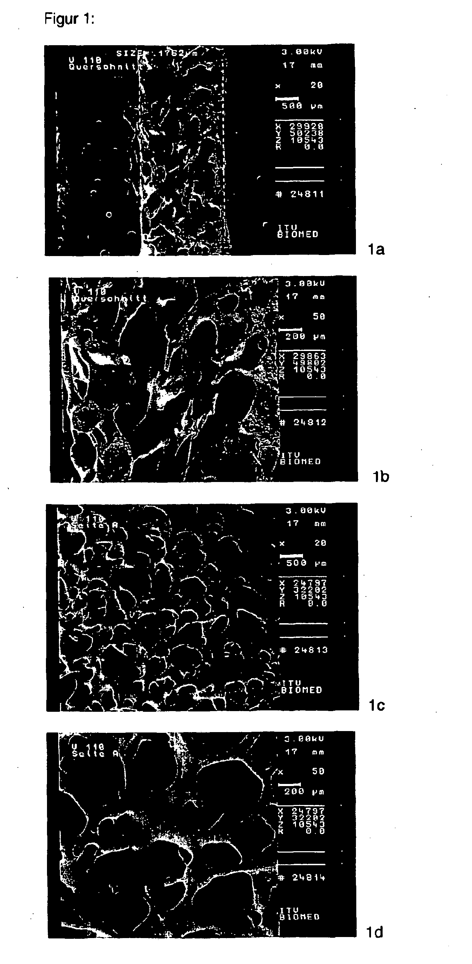

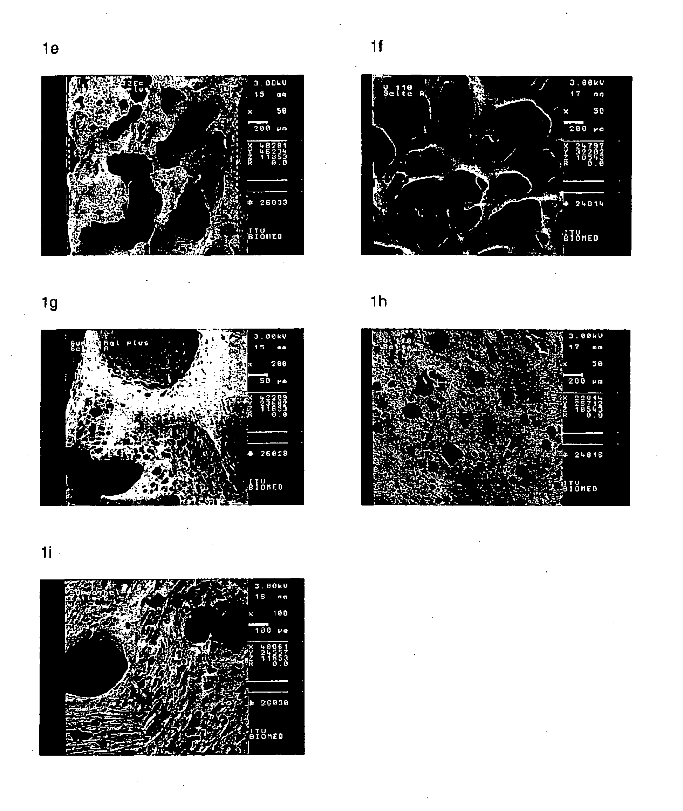

- Figur 1a-i:: REM-Aufnahmen eines undotierten Strukturschaums aus einem Terpolymer aus den Monomeren Lactid, ε-Capro- lacton und Trimethylencarbonat in vier unterschiedlichen Vergrößerungen,

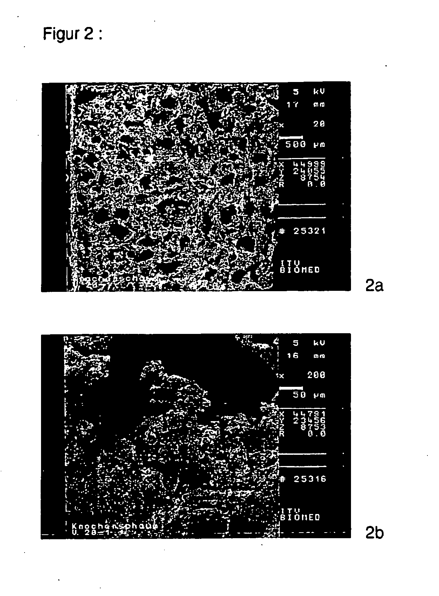

- Figur 2a-b:: REM-Aufnahmen eines mit TCP dotierten Strukturschaums aus einem Copolymer aus den Monomeren Lactid und Tri- methylencarbonat in zwei unterschiedlichen Vergrößerun- gen,

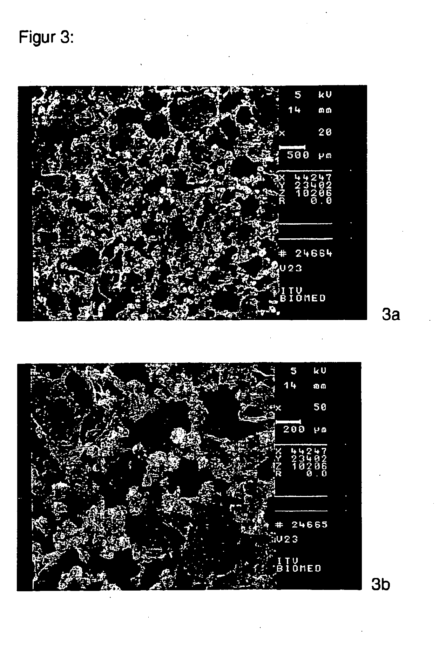

- Figur 3a-b:: REM-Aufnahmen eines mit Collagen dotierten Struktur- schaums aus einem Terpolymer aus den Monomeren Lac- tid, ε-Caprolacton und Trimethylencarbonat in zwei unter- schiedlichen Vergrößerungen.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

### Beispiel 1: Herstellung des Terpolymers aus Lactid, ε-Caprolacton und Trimethylencarbonat

Zur Herstellung des Terpolymers werden 1.500 g DL-Lactid, 200 g ε-Caprolacton und 300 g Trimethylencarbonat unter Rühren vermischt. Nach Zugabe des Katalysators (Zinnoctoat: 0,4 g entspricht 0,02 Gew.-%, bezogen auf das Gesamtgewicht des Ansatzes) wird unter Rühren auf 180°C erhitzt und 24 Stunden bei dieser Temperatur weiterpolymerisiert. Zum Ablassen des Reaktionsgemisches wird die Temperatur auf 200 °C erhöht, das Polymer ausgetragen und nach dem Erkalten auf eine Korngröße 5 mm gemahlen. Die Viskosität des Polymergranulats beträgt 1,32 dl/g, bezogen auf eine 0,1 %ige Lösung des Terpolymers in Chloroform bei 25 °C.

### Beispiel 2: Herstellung eines Copolymers aus Lactid und Trimethylencarbonat

Zur Herstellung des Copolymers werden 1.800 g DL-Lactid und 200 g Trimethylencarbonat unter Rühren vermischt. Nach Zugabe des Katalysators (Zinnoctoat: 0,4 g entspricht 0,02 Gew.-%, bezogen auf das Gesamtgewicht des Ansatzes) wird unter Rühren auf 170 °C erhitzt und 24 Stunden bei dieser Temperatur weiterpolymerisiert. Zum Ablassen des Reaktionsgemisches wird die Temperatur auf 180 bis 200 °C erhöht, das Polymer ausgetragen und nach dem Erkalten auf eine Korngröße von 5 mm gemahlen. Die Viskosität des granulierten Polymers beträgt 1,41 dl/g, bezogen auf eine 0,1 %ige Lösung des Copolymers in Chloroform bei 25 °C.

### Beispiel 3: Schaumherstellung ohne Füllmaterial (diskontinuierliches Verfahren)

In einem Glasreaktor werden 200 g granuliertes Polymer aus Beispiel 1 mit 1600 g Dimethylsulfoxid (DMSO) versetzt und unter Rühren eine ca. 11 %ige Polymerlösung hergestellt. Dieser Lösung werden 1.800 g Zucker (mittlere Korngröße 200 µm) portionsweise zugesetzt und innerhalb von 5 Minuten innig verrührt. Die erhaltene Polymersuspension wird in einer Stahlwanne 5 mm hoch ausgegossen und sofort auf -20°C eingefroren und anschließend 2 Stunden lang bei -20 °C belassen. Die eingefrorene Lösung wird zusammen mit der Stahlwanne in ein 5 l Fällbad mit 20 °C bis 30 °C warmen bidestillierten Wasser gegeben, wobei das Fällbad zur Beschleunigung der Lösung des Zuckers gerührt werden kann. Eine deutliche Beschleunigung der Schaumstoffbildung bzw. des Ausfäll- und Auswaschvorganges erzielt man mit einer Wasser-Umwälzapparatur oder Gegenstrom-Anlage. Der Schaumstoffkörper wird anschließend bis zur Gewichtskonstanz im Vakuum getrocknet. Nach dem Trocknen kann der Formkörper auf seine gewünschte Größe zugeschnitten werden, beispielsweise mit einem Skalpell oder Fällmesser.

| | |
|---|---|
| Ermittelte Daten: | |
| Schichtdicke: | 2 mm |
| Porosität: | 90 bis 94 % |
| Porengröße: | 50 bis 500 µm, interkonnektierend |
| Dichte: | 0,112 g/cm³ |
| Dehnung: | 60 bis 70 % - |
| Wasseraufnahmevermögen: | 1100 % (11-fache des Eigengewichtes) |

### Beispiel 4: Schaumherstellung mit TCP als Füllstoffmaterial (diskontinuierliches Verfahren)

In einem Glasreaktor werden 200 g granuliertes Polymer aus Beispiel 2 mit 1600 g Dimethylsulfoxid (DMSO) versetzt und unter Rühren eine ca. 11 %ige Polymerlösung hergestellt. Dieser Lösung werden 1.800 g Zucker und 200 g Tricalciumphosphat-Pulver (TCP-Pulver, mittlere Korngröße 200 µm) portionsweise zugesetzt und innerhalb von 5 Minuten innig verrührt. Die erhaltene Polymersuspension wird auf eine Stahlwanne 5 mm hoch ausgegossen und sofort auf -20 °C eingefroren und 2 Stunden bei - 20°C belassen. Die eingefrorene Lösung wird zusammen mit der Stahlwanne in ein 5 l Fällbad mit 20°C bis 30°C warmen bidestillierten Wasser gegeben, wobei das Fällbad zur Beschleunigung der Lösung des Zuckers gerührt werden kann.

Eine deutliche Beschleunigung der Schaumstoffbildung bzw. des Ausfäll- und Auswaschvorgangs erzielt man mit einer Wasser-Umwälzapparatur oder Gegenstrom-Anlage. Der Schaumstoffkörper wird anschließend bis zur Gewichtskonstanz im Vakuum getrocknet. Nach dem Trocknen kann der Formkörper auf seine gewünschte Größe zugeschnitten werden, beispielsweise mit einem Skalpell oder Fällmesser.

| | |
|---|---|
| Ermittelte Daten: | |
| Schichtdicke: | 2,5 mm |
| Porosität: | 84 bis 88 % |
| Porengröße: | 30 bis 400 µm, interkonnektierend |
| Dichte: | 0,205 g/cm³ |
| Dehnung: | 3 bis 5 % |
| Wasseraufnahmevermögen: | 810 % (8-fache des Eigengewichtes) |

### Beispiel 5: Schaumherstellung mit Collagen als Füllmaterial (diskontinuierliches Verfahren)

In einem Glasreaktor werden 200 g granuliertes Polymer aus Beispiel 1 mit 1.600 g Dimethylsulfoxid (DMSO) versetzt und unter Rühren eine ca. 11 %ige Polymerlösung hergestellt. Dieser Lösung werden 1.800 g Zucker und 50 g Collagen-Pulver (Korngröße < 50 µm) portionsweise zugesetzt und innerhalb von 5 Minuten innig verrührt.

Die erhaltene Polymersuspension wird auf eine Stahlwanne 5 mm hoch ausgegossen und sofort auf -20 °C eingefroren und 2 Stunden bei - 20°C belassen. Die eingefrorene Lösung wird zusammen mit der Stahlwanne in ein 5 l Fällbad mit 20°C bis 30°C warmen bidestillierten Wasser gegeben, wobei es sich empfiehlt, daß das Fällbad zur Beschleunigung der Lösung des Zuckers gerührt wird.

Eine deutliche Beschleunigung der Schaumstoffbildung bzw. des Ausfäll- und Auswaschvorgangs erzielt man mit einer Wasser-Umwälzapparatur oder Gegenstrom-Anlage. Der Schaumstoffkörper wird anschließend bis zur Gewichtskonstanz im Vakuum getrocknet. Nach dem Trocknen kann der Formkörper auf seine gewünschte Größe zugeschnitten werden, beispielsweise mit einem Skalpell oder Fällmesser.

| | |
|---|---|
| Ermittelte Daten: | |
| Schichtdicke: | 2,5 mm |
| Porosität: | 84 bis 88 % |
| Porengröße: | 30 bis 500 µm, interkonnektierend |
| Dichte: | 0,153 g/cm³ |
| Dehnung: | 40 bis 50 % |
| Wasseraufnahmevermögen: | 950 % (9,5-fache des Eigengewichtes) |

## Patentansprüche

1. Resorbierbarer und poröser Formkörper zur medizinischen Behandlung von Wunden, wobei er als Schaumstruktur vorliegt, die ein Co- und/oder Terpolymer auf der Basis der Monomere Lactid, Trimethylencarbonat, ε-Caprolacton und/oder Dioxan-2-on umfasst, **dadurch gekennzeichnet, dass** die Schaumstruktur in Form eines Strukturschaums mit einem Gerüst vorliegt, das Hohlräume umfasst, die Schaumstruktur eine Über- und eine Unterstruktur aufweist, wobei die Unterstruktur **dadurch** gebildet ist, dass das Material des Gerüstes selbst eine Schaumstruktur besitzt, wobei die Überstruktur eine Porengröße im Bereich von 50 bis 800 µm und die Unterstruktur eine Porengröße im Bereich von 0,1 bis 50 µm aufweist.

2. Formkörper nach Anspruch 1, **dadurch gekennzeichnet, dass** die Überstruktur eine Porengröße im Bereich von 80 bis 500 µm aufweist.

3. Formkörper nach Anspruch 1 oder 2, **dadurch gekennzeichnet, dass** die Unterstruktur eine Porengröße im Bereich 0,5 bis 30 µm aufweist.

4. Formkörper nach einem der vorhergehenden Ansprüche, **dadurch gekennzeichnet, daß** er interkonnektierende Poren aufweist.

5. Formkörper nach einem der vorhergehenden Ansprüche, **dadurch gekennzeichnet, dass** er eine Aufnahmekapazität für Flüssigkeiten aufweist, die dem 7- bis 15-fachen, insbesondere dem 8- bis 12-fachen, seines Eigengewichtes entspricht.

6. Formkörper nach einem der vorhergehenden Ansprüche, **dadurch gekennzeichnet, daß** er eine Dichte von maximal 0,3 g/cm³, vorzugsweise von weniger als 0,25 g/cm³, aufweist.

7. Formkörper nach einem der vorhergehenden Ansprüche, **dadurch gekennzeichnet, dass** er plastisch verformbar, insbesondere komprimierbar, dehnbar und/oder biegbar, ist.

8. Formkörper nach einem der vorhergehenden Ansprüche, **dadurch gekennzeichnet, dass** er ein Elastizitätsmodul von weniger als 2000 N/mm², vorzugsweise von weniger als 1000 N/mm² aufweist.

9. Formkörper nach einem der vorhergehenden Ansprüche, **dadurch gekennzeichnet, dass** er schneidbar ist.

10. Formkörper nach einem der vorhergehenden Ansprüche, **dadurch gekennzeichnet, dass** er eine Schichtdicke von 300 µm bis 30 cm aufweist.

11. Formkörper nach einem der vorhergehenden Ansprüche, **dadurch gekennzeichnet, dass** er als Blockstruktur, insbesondere mit einer Schichtdicke zwischen 5 und 30 cm, vorliegt.

12. Formkörper nach einem der vorhergehenden Ansprüche, **dadurch gekennzeichnet, dass** er als Flächengebilde, insbesondere als Flachstruktur, vorzugsweise mit einer Schichtdicke zwischen 300 µm und 5 cm, vorliegt.

13. Formkörper nach einem der vorhergehenden Ansprüche, **dadurch gekennzeichnet, dass** er einen Gehalt an freien Monomeren von 0 bis 10 Gew-%, insbesondere 0,1 bis 10 Gew.-%, vorzugsweise 1 bis 10 Gew.-%, bezogen auf das Gewicht des Co- und/oder Terpolymers, aufweist.

14. Formkörper nach einem der vorhergehenden Ansprüche, **dadurch gekennzeichnet, dass** das Co-und/oder Terpolymer mit einem Füllmaterial, insbesondere einem synthetischen Polymer, vorzugsweise Polyvinylalkohol (PVA), dotiert ist.

15. Formkörper nach Anspruch 14, **dadurch gekennzeichnet, dass** das Co- und/oder Terpolymer einen Gehalt an Füllstoffmaterial im Bereich zwischen 10 und 300 Gew.-%, vorzugsweise zwischen 20 und 100 Gew.-%, bezogen auf das Gesamtgewicht des Formkörpers, aufweist.

16. Verfahren zur Herstellung eines resorbierbaren und porösen Formkörpers, eines solchen nach einem der vorhergehenden Ansprüche, zur medizinischen Behandlung als Wundauflage, umfassend die Schritte:
- Herstellung einer Lösung eines Co- und/oder Terpolymers in einem ersten Lösungsmittel,
- Herstellung einer Suspension durch Zugabe eines in dem ersten Lösungsmittel nicht löslichen Stoffes zur Lösung,
- Abkühlung und Verfestigung der Suspension,
- Ausfällung des Co-und/oder Terpolymers und Herauslösung des Stoffes mit einem mit dem ersten Lösungsmittel mischbaren zweiten Lösungsmittel, das kein Lösungsmittel für das Co- und/oder Terpolymer ist.

17. Verfahren nach Anspruch 16, **dadurch gekennzeichnet, dass** als erstes Lösungsmittel mindestens ein organisches, insbesondere wasserlösliches, Lösungsmittel, vorzugsweise Dimethylsulfoxid (DMSO), Dimethylacetamid (DMA), Dimethylformamid (DMF), Tetrahydrofuran (THF), Dioxan oder Mischungen davon, verwendet wird.

18. Verfahren nach Anspruch 16 oder 17, **dadurch gekennzeichnet, dass** als zweites Lösungsmittel Wasser verwendet wird.

19. Verfahren nach einem der Ansprüche 16 bis 18, **dadurch gekennzeichnet, dass** als Stoff ein feinteiliger Stoff, vorzugsweise fester Stoff, insbesondere mit einer Korngröße von 50 bis 500 µm, insbesondere von 80 bis 500 µm, vorzugsweise mit einer mittleren Korngröße von ca. 200 µm, hinzugegeben wird.

20. Verfahren nach einem der Ansprüche 16 bis 19, **dadurch gekennzeichnet, dass** als Stoff ein wasserlöslicher Stoff, vorzugsweise Zucker, verwendet wird.

21. Verfahren nach einem der Ansprüche 16 bis 20, **dadurch gekennzeichnet, dass** als Füllmaterial ein im zweiten Lösungsmittel nicht löslicher Feststoff, insbesondere ein synthetisches Polymer, vorzugsweise Polyvinylalkohol (PVA), zur Suspension hinzugegeben wird.

22. Verfahren nach einem der Ansprüche 16 bis 21, **dadurch gekennzeichnet, dass** die Suspension bei einer Temperatur zwischen - 10 und - 30°C, insbesondere bei einer Temperatur von ca. - 20 °C, verfestigt wird.

23. Verfahren nach einem der Ansprüche 16 bis 22, **dadurch gekennzeichnet, dass** die Ausfällung und Herauslösung in einem wässrigen Fällbad, insbesondere in einem solchen mit einer Wassertemperatur zwischen 20 und 30 °C, vorgenommen wird.

24. Formkörper, herstellbar nach einem Verfahren nach einem der Ansprüche 16 bis 23.

25. Formkörper nach einem der vorhergehenden Ansprüche zur verwendung als Wundauflage bei der medizinischen Behandlung von Mensch und/oder Tier.

26. Formkörper nach einem der vorhergehenden Ansprüche zur verwendung als Hautersatzmaterial bei der medizinischen Behandlung von Mensch und/oder Tier.

27. Formkörper nach einem der vorhergehenden Ansprüche zur verwendung als Adhäsionsprophylaxe bei der medizinischen Behandlung von Mensch und/oder Tier.

28. Formkörper nach einem der vorhergehenden Ansprüche zur Verwendung als Matrix für Zellkulturen.

## Claims

1. Absorbable and porous shaped article for the medical treatment of wounds, said article being in the form of a foam structure which includes a co- and/or terpolymer based on the monomers lactide, trimethylene carbonate, ε-caprolactone and/or dioxan-2-one, **characterized in that** the foam structure is in the form of a structural foam with a framework which includes voids, the foam structure includes a superstructure and a substructure, wherein the substructure is formed through the material of the framework itself having a foam structure, wherein the superstructure has a pore size in the range from 50 to 800 µm and the substructure has a pore size in the range from 0.1 to 50 µm.

2. Shaped article according to Claim 1, **characterized in that** the superstructure has a pore size in the range from 80 to 500 µm.

3. Shaped article according to Claim 1 or 2, **characterized in that** the substructure has a pore size in the range from 0.5 to 30 µm.

4. Shaped article according to any of the preceding claims, **characterized in that** it has interconnecting pores.

5. Shaped article according to any of the preceding claims, **characterized in that** it has an absorption capacity for liquids which is equivalent to 7 to 15 times, in particular 8 to 12 times, its own weight.

6. Shaped article according to any of the preceding claims, **characterized in that** it has a density not exceeding 0.3 g/cm³, preferably of less than 0.25 g/cm³.

7. Shaped article according to any of the preceding claims, **characterized in that** it is plastically deformable, in particular compressible, extensible and/or bendable.

8. Shaped article according to any of the preceding claims, **characterized in that** it has a modulus of elasticity of less than 2000 N/mm², preferably of less than 1000 N/mm².

9. Shaped article according to any of the preceding claims, **characterized in that** it is cuttable.

10. Shaped article according to any of the preceding claims, **characterized in that** it has a layer thickness of from 300 µm to 30 cm.

11. Shaped article according to any of the preceding claims, **characterized in that** it is in the form of a block structure, in particular with a layer thickness of between 5 and 30 cm.

12. Shaped article according to any of the preceding claims, **characterized in that** it is in the form of a sheet-like material, in particular of a flat structure, preferably with a layer thickness of between 300 µm and 5 cm.

13. Shaped article according to any of the preceding claims, **characterized in that** it has a free monomer content of from 0 to 10% by weight, in particular 0.1 to 10% by weight, preferably 1 to 10% by weight, based on the weight of the co- and/or terpolymer.

14. Shaped article according to any of the preceding claims, **characterized in that** the co- and/or terpolymer is doped with a filling material, in particular a synthetic polymer, preferably polyvinyl alcohol (PVA).

15. Shaped article according to Claim 14, **characterized in that** the co- and/or terpolymer has a content of filling material in the range between 10 and 300% by weight, preferably between 20 and 100% by weight, based on the total weight of the shaped article.

16. Process for producing an absorbable and porous shaped article, one according to any of the preceding claims, for medical treatment as wound-contact material, including the steps:
- preparation of a solution of a co- and/or terpolymer in a first solvent,
- preparation of a suspension by adding a substance which is insoluble in the first solvent to the solution,
- cooling and solidification of the suspension,
- precipitation of the co- and/or terpolymer and dissolving out of the substance with a second solvent which is miscible with the first solvent but which is not a solvent for the co- and/or terpolymer.

17. Process according to Claim 16, **characterized in that** at least one organic, in particular water-soluble, solvent, preferably dimethyl sulfoxide (DMSO), dimethylacetamide (DMA), dimethylformamide (DMF), tetrahydrofuran (THF), dioxane or mixtures thereof is used as first solvent.

18. Process according to Claim 16 or 17, **characterized in that** water is used as second solvent.

19. Process according to any of Claims 16 to 18, **characterized in that** the substance added is a fine-particle substance, preferably solid substance, in particular with a particle size of from 50 to 500 µm, in particular from 80 to 500 µm, preferably with an average particle size of about 200 µm.

20. Process according to any of Claims 16 to 19, **characterized in that** a water-soluble substance, preferably sugar, is used as substance.

21. Process according to any of Claims 16 to 20, **characterized in that** a solid which is insoluble in the second solvent, in particular a synthetic polymer, preferably polyvinyl alcohol (PVA), is added as filling material to the suspension.

22. Process according to any of Claims 16 to 21, **characterized in that** the suspension is solidified at a temperature of between -10 and -30°C, in particular at a temperature of about -20°C.

23. Process according to any of Claims 16 to 22, **characterized in that** the precipitation and dissolving out is carried out in an aqueous precipitating bath, in particular in one with a water temperature of between 20 and 30°C.

24. Shaped article which can be produced by a process according to any of Claims 16 to 23.

25. Shaped article according to any of the preceding claims for use as wound-contact material in the medical treatment of humans and/or animals.

26. Shaped article according to any of the preceding claims for use as skin substitute material in the medical treatment of humans and/or animals.

27. Shaped article according to any of the preceding claims for use as adhesion prophylaxis in the medical treatment of humans and/or animals.

28. Shaped article according to any of the preceding claims for use as matrix for cell cultures.

## Revendications

1. Corps moulé résorbable et poreux destiné au traitement médical de plaies, se trouvant sous forme de structure de mousse, qui comprend un copolymère et/ou un terpolymère à base des monomères lactide, triméthylènecarbonate, ε-caprolactone et/ou dioxan-2-one, **caractérisé en ce que** la structure de mousse se trouve sous forme d'une mousse de structure avec une charpente, qui comprend des espaces creux, la structure de mousse présente une sur-structure et une sous-structure, où la sous-structure est formée en raison du fait que le matériau de la charpente présente lui-même une structure de mousse et où la sur-structure présente une grosseur des pores dans la plage de 50 à 800 µm et la sous-structure présente une grosseur des pores dans la plage de 0,1 à 50 µm.

2. Corps moulé selon la revendication 1, **caractérisé en ce que** la sur-structure présente une grosseur des pores dans la plage de 80 à 500 µm.

3. Corps moulé selon la revendication 1 ou 2, **caractérisé en ce que** la sous-structure présente une grosseur des pores dans la plage de 0,5 à 30 µm.

4. Corps moulé selon l'une quelconque des revendications précédentes, **caractérisé en ce qu'**il présente des pores interconnectés.

5. Corps moulé selon l'une quelconque des revendications précédentes, **caractérisé en ce qu'**il présente une capacité d'absorption pour des liquides qui correspond à 7 jusqu'à 15 fois, en particulier à 8 jusqu'à 12 fois son propre poids.

6. Corps moulé selon l'une quelconque des revendications précédentes, **caractérisé en ce qu'**il présente une densité d'au maximum 0,3 g/cm³, de préférence de moins de 0,25 g/cm³.

7. Corps moulé selon l'une quelconque des revendications précédentes, **caractérisé en ce qu'**il est plastiquement déformable, en particulier comprimable, extensible et/ou flexible.

8. Corps moulé selon l'une quelconque des revendications précédentes, **caractérisé en ce qu'**il présente un module d'élasticité de moins de 2000 N/mm², de préférence de moins de 1000 N/mm².

9. Corps moulé selon l'une quelconque des revendications précédentes, **caractérisé en ce qu'**il peut être découpé.

10. Corps moulé selon l'une quelconque des revendications précédentes, **caractérisé en ce qu'**il présente une épaisseur de couche de 300 µm à 30 cm.

11. Corps moulé selon l'une quelconque des revendications précédentes, **caractérisé en ce qu'**il se trouve sous forme de structure à blocs, en particulier d'une épaisseur de couche entre 5 et 30 cm.

12. Corps moulé selon l'une quelconque des revendications précédentes, **caractérisé en ce qu'**il se trouve sous forme d'entité étendue, en particulier sous forme de structure plane, présentant de préférence une épaisseur de couche entre 300 µm et 5 cm.

13. Corps moulé selon l'une quelconque des revendications précédentes, **caractérisé en ce qu'**il présente une teneur en monomères libres de 0 à 10% en poids, en particulier de 0,1 à 10% en poids, de préférence de 1 à 10% en poids, par rapport au poids du copolymère et/ou du terpolymère.

14. Corps moulé selon l'une quelconque des revendications précédentes, **caractérisé en ce que** le copolymère et/ou le terpolymère est dopé avec un matériau de remplissage, en particulier un polymère synthétique, de préférence un poly(alcool vinylique) (PVA).

15. Corps moulé selon la revendication 14, **caractérisé en ce que** le copolymère et/ou le terpolymère présente une teneur en matériau de remplissage dans la plage de 10 à 300% en poids, de préférence de 20 à 100% en poids, par rapport au poids total du corps moulé.

16. Procédé pour la production d'un corps moulé résorbable et poreux, selon l'une quelconque des revendications précédentes, destiné à un traitement médical, comme pansement pour plaies, comprenant les étapes suivantes :
- préparation d'une solution d'un copolymère et/ou d'un terpolymère dans un premier solvant,
- préparation d'une suspension par addition à la solution d'une substance non soluble dans le premier solvant,
- refroidissement et solidification de la suspension,
- précipitation du copolymère et/ou du terpolymère et séparation par dissolution de la substance avec un deuxième solvant qui n'est pas un solvant pour le copolymère et/ou le terpolymère, miscible avec le premier solvant.

17. Procédé selon la revendication 16, **caractérisé en ce qu'**on utilise comme premier solvant au moins un solvant organique, en particulier soluble dans l'eau, de préférence le diméthylsulfoxyde (DMSO), le diméthylacétamide (DMA), le diméthylformamide (DMF), le tétrahydrofurane (THF), le dioxane ou leurs mélanges.

18. Procédé selon la revendication 16 ou 17, **caractérisé en ce qu'**on utilise de l'eau comme deuxième solvant.

19. Procédé selon l'une quelconque des revendications 16 à 18, **caractérisé en ce qu'**on ajoute comme substance une substance finement divisée, de préférence une substance solide, présentant en particulier une grosseur des grains de 50 à 500 µm, en particulier de 80 à 500 µm, de préférence une grosseur moyenne d'environ 200 µm.

20. Procédé selon l'une quelconque des revendications 16 à 19, **caractérisé en ce qu'**on utilise comme substance une substance soluble dans l'eau, de préférence du sucre.

21. Procédé selon l'une quelconque des revendications 16 à 20, **caractérisé en ce qu'**on ajoute à la suspension comme matériau de remplissage un solide non soluble dans le deuxième solvant, en particulier un polymère synthétique, de préférence un poly(alcool vinylique) (PVA).

22. Procédé selon l'une quelconque des revendications 16 à 21, **caractérisé en ce que** la suspension est solidifiée à une température entre -10 et -30°C, en particulier à une température d'environ -20°C.

23. Procédé selon l'une quelconque des revendications 16 à 22, **caractérisé en ce que** la précipitation et la séparation par dissolution sont réalisées dans un bain de précipitation aqueux, en particulier dans un bain présentant une température de l'eau entre 20 et 30°C.

24. Corps moulé, pouvant être préparé selon un procédé selon l'une quelconque des revendications 16 à 23.

25. Corps moulé selon l'une quelconque des revendications précédentes destiné à une utilisation comme pansement lors du traitement médical d'un homme et/ou d'un animal.

26. Corps moulé selon l'une quelconque des revendications précédentes destiné à une utilisation comme matériau de remplacement de la peau lors du traitement médical d'un homme et/ou d'un animal.

27. Corps moulé selon l'une quelconque des revendications précédentes destiné à une utilisation comme prophylaxie d'adhérence lors du traitement médical d'un homme et/ou d'un animal.

28. Corps moulé selon l'une quelconque des revendications précédentes destiné à une utilisation comme matrice pour des cultures cellulaires.
